(19) Europäisches Patentamt / European Patent Office / Office européen des brevets

(11) **EP 3 270 157 B1**

(12) **EUROPEAN PATENT SPECIFICATION**

(45) Date of publication and mention
of the grant of the patent:
**26.01.2022  Bulletin 2022/04**

(21) Application number: **16178864.1**

(22) Date of filing: **11.07.2016**

(51) International Patent Classification (IPC):
*G01N 33/50* (2006.01)      *G01N 33/566* (2006.01)

(52) Cooperative Patent Classification (CPC):
**G01N 33/566; G01N 33/5008;** G01N 2333/726;
G01N 2333/938; G01N 2500/00

(54) **SCREENING ASSAY FOR SWEETNESS**

SCREENING-ASSAY FÜR SÜSSE

ESSAI DE CRIBLAGE DE SUCROSITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.01.2018  Bulletin 2018/03**

(73) Proprietor: **Firmenich SA**
**1242 Satigny (CH)**

(72) Inventors:
• **PAQUES, Marcel**
  **6700 AE Wageningen (NL)**
• **BASTIAAN-NET, Shanna**
  **6700 AE Wageningen (NL)**
• **MES, Jurriaan, Johannes**
  **6700 AE Wageningen (NL)**
• **KERSCH-COUNET, Christine**
  **6700 AE Wageningen (NL)**

(74) Representative: **Strych, Sebastian**
**Mitscherlich PartmbB**
**Patent- und Rechtsanwälte**
**Sonnenstraße 33**
**80331 München (DE)**

(56) References cited:
WO-A1-01/59451          WO-A1-2011/067202
WO-A1-2012/178079     WO-A2-2004/065963
WO-A2-2005/031309

## Description

### Field of the Invention

The present invention refers to a method for testing the activation of a G-protein coupled taste receptor being a sweet, umami or bitter taste receptor comprising the subunits T1R1, T1R2 T1R3 and/or T2Rs in different combinations which are used for example for the screening of a sweet taste modulating compound. The compound is tested on a cell natively expressing the taste receptor and comprising an enzyme complementation system. Moreover, the invention is directed to a kit for screening sweet, umami and/or bitter taste modulating compounds or mixtures thereof for example in product- and model-matrices (e.g. buffer etc.).

### Background of the invention

The perception of taste is mediated by G protein-coupled receptors: For example the heterodimeric receptor T1R2/T1R3 is sensitive to sweetness, the heterodimeric receptor T1R1/T1R3 is sensitive to umami taste and the T2R class of G-protein receptors are sensitive to bitter taste (Li, 2009). Taste receptors are for example present in taste bud cells (Hoon et al 1999; Adler et al 2000; Nelson et al 2001), but also in the gut (Kojima et al., 2014). In addition to taste, the sweet receptor T1R2/T1R3 for example is suggested to play a role in many other regulatory processes such as sugar uptake and energy metabolism in different organs and cell types (Nakagawa et al. 2009; Ren et al. 2009; Elliott et al. 2011; Haid et al. 2011; Kyriazis et al. 2012; Gravina et al. 2013). *In vitro* cell assays, transiently expressing this taste receptor in cell lines, either with or without interacting G-proteins, are an important research tool to obtain a fundamental understanding of taste perception but also form an important tool to identify new low-caloric sugar replacers and enhancers/inhibitors. Fujiwara et al. (2012) showed that the sweet enhancing characteristics of neohesperidin dihydrocholcone (NHDC) and cyclamate reported in human sensory evaluation, were also observed in receptor-based cell assays. Thus, such cell-based assays also provide important tools for compound library screening which has led to the discovery, exploration and subsequent commercialization of selective enhancers of sucralose and sucrose (Servant et al. 2010; Servant et al. 2011).

GPCR (G-protein coupled receptor) activation responses in *in vitro* cell assays can be monitored by determining the direct conformational changes upon agonist binding, either of the receptor itself (Hoffmann et al. 2008) or of its interacting heterotrimeric G proteins (Chung et al. 2011) and beta-arrestins (Xiao et al. 2004; Shukla et al. 2008). Also the release of two downstream second messengers, cyclic AMP (cAMP) or inositol 1,4,5-trisphosphate (IP3), resulting from such conformational changes, can be measured using ELISA. Alternatively, subsequent release of intracellular calcium ($Ca^{2+}$) and membrane depolarisation can be monitored using calcium or voltage sensitive dyes (Paredes et al. 2008; Fairless et al. 2013). WO 2012/178079, finally, discloses the monitoring of protein trafficking in a cell using beta-galactosidase reporter fragments.

Interestingly, reduced $Ca^{2+}$ influx responses were observed in T1R2/T1R3 expressing cells after successive stimulation of a tastant (Lefkowitz et al. 1992), while full response recovery was regained after a period of 5 min between successive stimulations (Nelson at al. 2001) pointing towards the existence of a mechanism for receptor desensitisation and recycling. However, how taste receptors are sensitized, such as the sweet (T1R2/T1R3) receptor, the umami (T1R1, T1R2 T1R3) receptor or bitter (T2Rs) receptors is thus far poorly understood. The majority of G-protein coupled receptors (GPCRs) utilize the beta-arrestin-dependent clathrin-mediated endocytosis pathway for receptor desensitisation, internalization and subsequent recycling or degradation (Claing et al. 2002; Reiter and Lefkowitz 2006). Typically, the carboxy-terminal tail of stimulated GPCRs are phosphorylated by protein-coupled receptor kinases (GRKs) upon agonist-induced G-protein receptor dissociation. As a consequence, beta-arrestin is recruited to the agonist-occupied receptor, serving as an adapter for the clathrin-endocytotic machinery. Once internalised, receptors are translocated to endosomal compartments to facilitate ligand detachment after which they are either shuttled back to the cell surface or to lysosomes where they are degraded by proteolytic activity (Lee et al. 2000; Sorkin and von Zastrow 2002).

Screening methods of the prior art such as the method described in US 7,927,825 for quantifying taste of compounds are performed on T1R2/T1R3 taste receptors stably or transiently, but not natively expressed in cells. WO 2011/067202 discloses methods for the screening of taste receptor modulators wherein the taste receptor is fused to a luminescent or fluorescent protein and is stably or transiently expressed in a cell. Further, prior art such as US 2010/0120063 describes enzyme assays for candidate compounds affecting G-protein coupled receptor activity based on enzyme complementation, and WO 2010/088633 describes a method for screening cells natively expressing a sweet taste receptor.

WO 2004/065963, WO 01/59451 and WO 2005/031309 provide further examples of screening assays using taste receptors.

So far, methods for screening taste compounds comprise mainly taste receptors which are stably or transiently expressed in a cell, in particular in an easily transfectable cell which does not natively express the transfected taste receptor. The disadvantage of these cells is that the taste receptor may not be expressed and/or folded properly, and the pathway as such may not function properly like in a native cell. Moreover, easily transfectable cells may not be the optimal system to provide a natural, optimal environment for a taste receptor. Activation of a taste receptor is often measured based on cAMP and/or $Ca^{2+}$ readout which represent very general responses of a cell on all kinds of changes and stimuli bearing the risk of a high number of false positive or negative results.

The present invention provides an improved method for testing the activation of a taste receptor and for screening a taste compound such as a sweet taste modulating compound, wherein a cell natively expressing a taste receptor is used in combination with an enzyme complementation system expressed in this cell which becomes active when a taste receptor is activated and for example internalized.

## Summary of the invention

The invention, in one aspect, presents a method for testing the activation of a G-protein coupled taste receptor comprising the steps of:

a) providing a cell natively expressing the taste receptor and comprising an enzyme complementation system,
b) adding a sweet-taste, umami-taste and/or bitter-taste modulating compound or mixtures of sweet, umami and/or bitter taste modulating compounds to the cell,
c) adding a substrate for an enzyme of the enzyme complementation system to the cell, wherein the substrate is reacting with the enzyme, when the taste receptor is activated, providing a signal, and
d) detecting the signal; wherein the G-protein coupled taste receptor is a sweet taste receptor comprising the subunits T1R2 and T1R3 (T1R2/ T1R3), an umami taste receptor comprising the subunits T1R1 and T1R3 (T1R1/T1R3), or any of the bitter taste receptors (T2Rs).

In another aspect, the invention resides in a method for screening a sweet taste modulating compound comprising subjecting this sweet taste modulating compound to a method according to the preceding paragraph, and subjecting the signal or signals detected in step d) to comparison with one or more reference compounds selected from the group consisting of standard compounds, other sweet taste modulating compounds, and/or combinations thereof.

In a further aspect, the invention provides a kit for testing the activation of a G-protein coupled taste receptor or for screening a taste modulating compound comprising a cell natively expressing the taste receptor and stably or transiently expressing an enzyme complementation system, and a substrate for the enzyme of the enzyme complementation system, wherein the receptor is a sweet taste receptor comprising the subunits T1R2 and T1R3 (T1R2/ T1R3), wherein the G-protein coupled taste receptor is an umami taste receptor comprising the subunits T1R1 and T1R3 (T1R1/T1R3), or wherein the G-protein coupled taste receptor is any of the bitter taste receptors (T2Rs).

## Figures

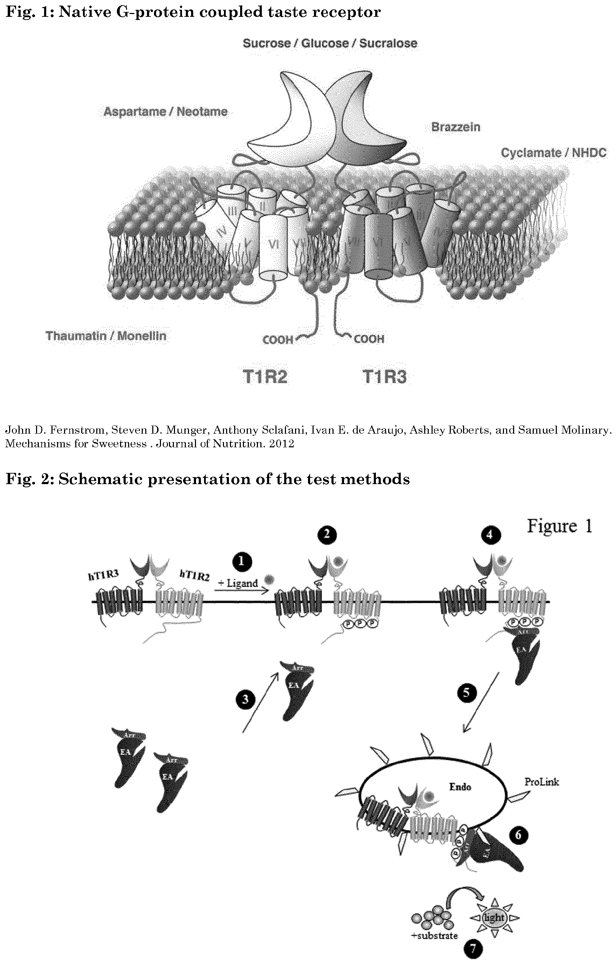

**Fig. 1** depicts a native G-protein coupled taste receptor for sweet taste consisting of the subunits T1R2 and T1R3 having binding sites natural and artificial sweet compounds such as sucrose, glucose, lactose, sucralose, aspartame, neotame, thaumatin, monelin, brazzein, cyclamate and NHDC. From John D. Fernstrom, Steven D. Munger, Anthony Sclafani, Ivan E. de Araujo, Ashley Roberts, and Samuel Molinary. Mechanisms for Sweetness . Journal of Nutrition. 2012.

**Fig. 2** schematically presents the method for testing the activation of a G-protein coupled taste receptor according to the present invention. For example the sweet taste receptor T1R2/T1R3 cell-assay is based on the native expression of the receptor in a cell and an enzyme complementation system such as PathHunter™ of for example DiscoveRx. Upon binding of a ligand such as a sweet taste modulating compound to the T1R2/T1R3 receptor dimer (1), receptor phosphorylation occurs, resulting in an activated T1R2/T1R3 receptor (2). Phosphorylation of ligand-bound receptors promotes beta-arrestin recruitment (3), formation of an arrestin-receptor complex (4) and subsequent endocytosis and trafficking of this complex to the endosome (5). A part of the enzyme such as beta-galactosidase (beta-Gal) enzyme acceptor (termed EA) fused to beta-arrestin is brought in close proximity to the other part of the enzyme such as beta-Gal ProLink™ enzyme localized on the endosome surface resulting in enzyme complementation of beta-Gal (6). Restored activity of beta-Gal can be quantified by reacting with a substrate of the enzyme for example with PathHunter™ Detection reagents (7).

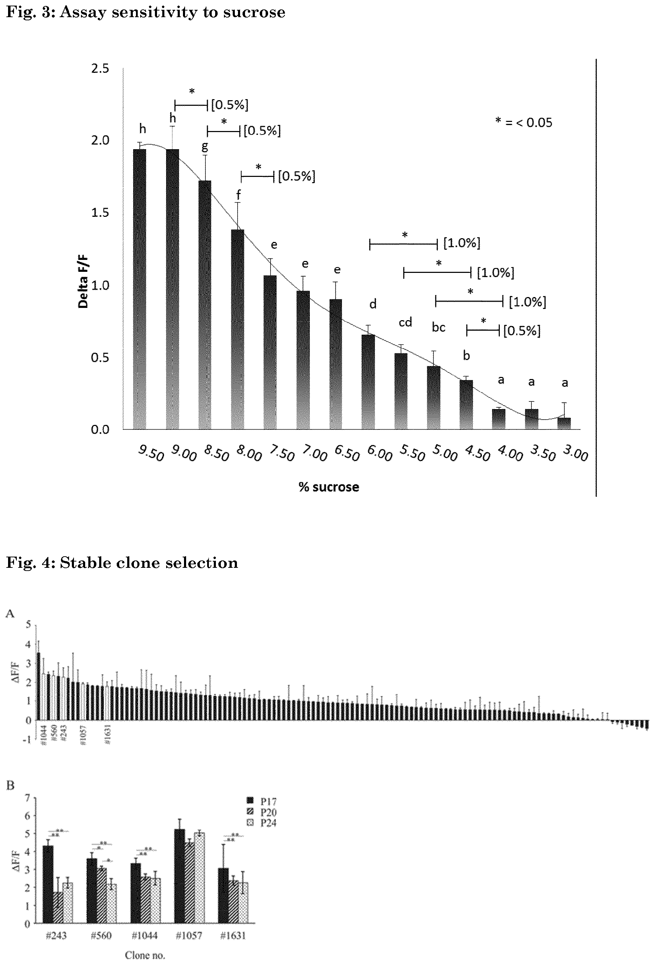

**Fig. 3** refers to the results of an assay for testing the sensitivity of the method of the present invention of 0.5 % for sucrose. The Simulated Milk Ultra Filtrate (SMUF) buffer has been prepared according to Jenness R. and Koops, J., Netherlands Milk Dairy J., 1962, p. 153-164). The sensitivity and significance was preferred to be 0.5% in this example since this correlates to a the ability of a trained sensory panel to determine the same differences. As is clear from Fig 3. , the sensitivity of 0.5% is obtained in the range 8 - 9% sucrose; from 5-6 % a sensitivity of 1% was obtained.

**Fig. 4** presents stable clone selection and validation of functional T1R2/T1R3 receptor expression. A) Receptor internalization responses of 141 single clones stimulated with 300 mM maltose in LG-DMEM (low glucose, Dulbecco's Modified Eagle Medium; mean $\pm$ Stdv; n=2). B) Stability of receptor internalization activities during cell passages was determined for clones #243, #560, #1044, #1057 and #1631. Beta-arrestin-induced receptor internalisation responses were quantified for 300 mM maltose during cell passages 17, 20 and 24 (mean $\pm$ Stdv; n=3).

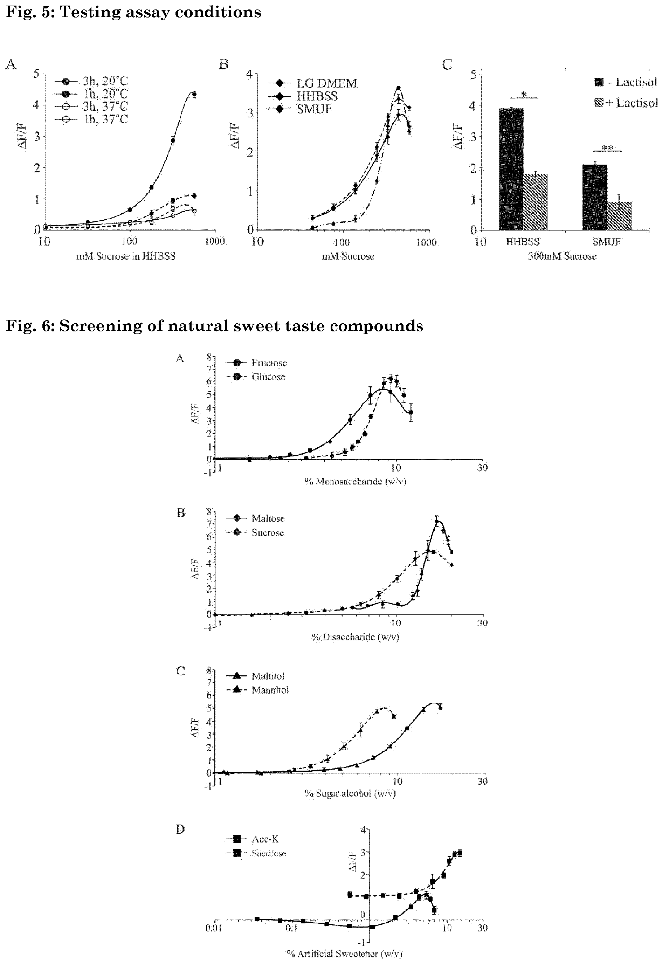

**Fig. 5** shows PathHunter™ assay conditions for optimal T1R2/T1R3 receptor internalisation responses. A) Optimal receptor internalisation responses for sucrose in HHBSS were obtained after 3 h of taste compound stimulation at 20°C. B) Dose-response curves of sucrose stimulation in LG DMEM (without FBS (fetal bovine serum)), HHBSS (Hank's buffered salt solution comprising HEPES (4-(2-hydroxyethyl)-1-piperazineethanesulfonic acid))) or SMUF (simulated milk ultra filtrate) buffer. C) Inhibition of receptor internalisation responses of 300 mM sucrose in HHBSS or SMUF buffer by 20 mM lactisole. Significance was determined by general ANOVA with Fishers Unprotected post-taste; *P < 0.001, **P < 0.002.

**Fig. 6** shows the screening of natural sweet taste compounds depicting beta-arrestin mediated-internalisation responses of PathHunter™HEK293 Active Internalisation Clone #1057 stimulated by monosaccharides (A), disaccharides (B), sugar alcohols (C) or artificial sweeteners (D). Receptor internalisation responses are expressed as the mean $\Delta F/F$ of three independent replicates. Stimulants are expressed as % (w/v) on a log10-scale. Ace-K: acesulfame-K.

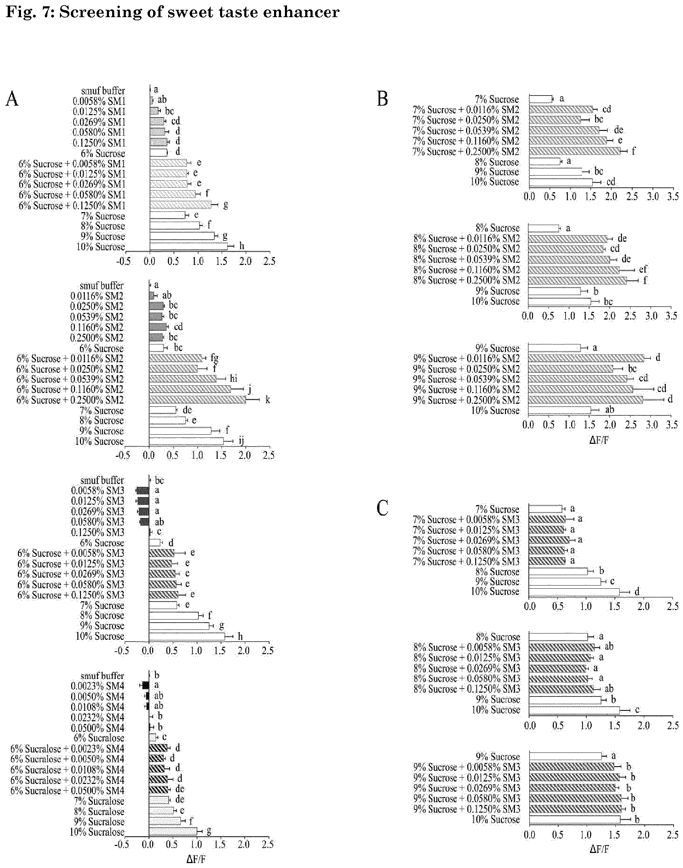

**Fig. 7** shows sweet receptor internalisation modulated by sweet taste enhancers. A) beta-arrestin-mediated T1R2/T1R3 internalisation by 6% sucrose or sucralose is increased when #1057 cells were simultaneously stimulated with different concentrations of the sweet modulators (SM) Modulasense (SM1), EN-336-983-7 (SM2), SweetGem (SM3) and SucralGem (SM4). B) The sweet modulator EN-336-983-7 (SM2) enhances receptor internalisation responses when combined with 7, 8 and 9% sucrose in a concentration dependent manner. C) Besides enhancing effects on 6% sucrose, Sweet Gem (SM3) also displayed increased receptor internalisation responses on 9% sucrose but not on 7 or 8% sucrose. Receptor internalisation responses are expressed as the mean $\Delta F/F$ of three independent replicates (n=3). Significance was determined by general ANOVA with Fishers Unprotected post-taste (p<0.05).

## Detailed description

The present invention refers to a method for testing the activation of a G-protein coupled taste receptor comprising the steps of:

a) providing a cell natively expressing the taste receptor and comprising an enzyme complementation system,
b) adding a taste modulating compound to the cell,
c) adding a substrate for an enzyme of the enzyme complementation system to the cell, wherein the substrate is reacting with the enzyme, when the taste receptor is activated, providing a signal, and
d) detecting the signal;

wherein the G-protein coupled taste receptor is a sweet taste receptor comprising the subunits T1R2 and T1R3 (T1R2/T1R3), an umami taste receptor comprising the subunits T1R1 and T1R3 (T1R1/T1R3), or any of the bitter taste receptors (T2Rs).

In an embodiment, the method is an *in vitro* method using cells such as HEK-293, Caco-2/TC7 or MIN6 cells or isolated primary cells.

The cells comprise an enzyme complementation system, wherein the enzyme is separated in two or more parts and each part is fused to a different component of a cell such as factors of the G-protein coupled receptor pathway, e.g., beta-arrestin and the endosome. Once a receptor is activated and in consequence the G-protein coupled receptor pathway is activated, factors of this pathway spatially come closer to each other and the different parts of the enzyme form a functional active enzyme which results in a very sensitive and specific testing and screening method, respectively. The enzyme can then react with a specific substrate that is degraded by the enzyme which results in a measurable signal. The enzyme complementation system is stably or transiently expressed in the cell.

In some embodiments the method for testing is used in a method for screening a sweet taste modulating compound such as a food product, a beverage, a medicament or extract thereof, a natural sugar such as lactose, sucrose, glucose, maltose or fructose, a non-caloric or low caloric sweetener, a sweet taste modulator such as an enhancer and/or an inhibitor, or a blend having enhanced or reduced sweet taste. Inhibitors of sweetness taste can be tested if the taste receptor is stimulated and an inhibitor is added.

Further, the present invention relates to a kit for testing the activation of a G-protein coupled taste receptor or for screening a taste modulating compound comprising a cell natively expressing the taste receptor and stably or transiently expressing an enzyme complementation system, and a substrate for the enzyme of the enzyme complementation system, wherein the receptor is a sweet taste receptor comprising the subunits T1R2 and T1R3 (T1R2/ T1R3), wherein the G-protein coupled taste receptor is an umami taste receptor comprising the subunits T1R1 and T1R3 (T1R1/T1R3), or wherein the G-protein coupled taste receptor is any of the bitter taste receptors (T2Rs).

The methods and the kit of the present invention allow to identify new sweet, umami or bitter taste modulating compounds or sweet, umami and/or bitter taste modulators, such as an inhibitor and/or an enhancer, as well as synergism and optimal combinations of mixtures, respectively. In addition, the method and kit are suitable for quality control of different batches comprising sweet, umami and/or bitter taste modulating compounds.

A taste receptor is a type of receptor which facilitates the sensation of taste. When food or other substances enter the mouth, molecules interact with saliva and are bound to taste receptors in the oral cavity and other locations. Molecules which give a sensation of taste are considered "sapid". Taste receptors are divided into two families Type 1, sweet and Type 2, bitter. Combinations of these receptors in dimers or other complexes contributes to different perceptions of taste. Visual, olfactive, "sapictive" (the perception of tastes), trigeminal (hot, cool), mechanical, all contribute to the perception of taste. The standard bitter, sweet, or umami taste receptor is a G protein-coupled receptor with seven transmembrane domains, while the salty and sour perceptions are probably mainly detected by ion channels. In 2015, scientists (Running et al, Chem. Senses (2015) 40 (7): 507-516) presented also the discovery of the sixth taste: the taste of fat or oleogustus. The fat taste receptors are also G protein-coupled receptors such as the GPR120 (G-protein coupled receptor 120). The present invention is not limited to sweet taste receptors, but all the embodiments described are likewise relevant for testing of bitter or umami taste modulating compounds on these bitter, or umami taste receptors located in the mouth and/or in the gut of human beings and animals, where also sweet taste receptors can be located alone or in combination with one or more of the other taste receptors.

Ligand binding at the taste receptors activate second messenger cascades to depolarize the taste cell. The T1R2 and T1R3 heterodimer receptor functions for example as the sweet receptor by binding to a wide variety of sugars and sugar substitutes. T1R2 and T1R3 expressing cells are found for example in circumvallate papillae and foliate papillae near the back of the tongue and palate taste receptor cells in the roof of the mouth, but also in several other types of organ tissue such the gut. These cells are shown to synapse upon the chorda tympani and glossopharyngeal nerves to send their signals to the brain. The T1R3 homodimer also functions as a sweet receptor in much the same way as T1R2 and T1R3 (T1R2/T1R3) but has decreased sensitivity to sweet substances.

The present invention makes - amongst others - use of the involvement of beta-arrestin in downstream signalling of a natively expressed taste receptor which is for example expressed in a HEK-293 cell comprising an enzyme complementation system such as the PathHunter™ GPCR technology, which is very sensitive. This system allows for example to monitor the beta-arrestin-mediated endosomal receptor internalisation activity. The present invention surprisingly shows that natural sugars such as lactose, fructose, glucose, sucrose, maltose, maltitol and mannitol and artificial sweeteners such as acesulfame-K and sucralose stimulate enzyme complementation activity in a concentration dependent manner. Further surprisingly it is shown that sugar induced cell responses are modified by sweetness enhancers, suggesting that T1R2/T1R3 receptor desensitisation and internalisation is mediated by beta-arrestin-induced endocytosis. Thus, the method for testing activation of a G-protein coupled taste receptor and its use for screening a sweet taste modulating compound allows effective screening for novel non-caloric or low caloric sweeteners, sweet taste modulators or optimal blends with enhanced sweet taste.

A sweet, umami or bitter taste modulating compound according to the present invention is any compound for oral intake modulating, e.g., inhibiting and/or enhancing, the sweet, umami or bitter taste of a compound, respectively, such as a food product, a beverage or a medicament or extract thereof, or any sweetening agent, umami or bitter taste mediating agent. A sweetening agent is for example a natural sugar (e.g., lactose, fructose, glucose, sucrose, maltose, maltitol and mannitol) or an artificial, e.g., non-caloric or low caloric sweetener (e.g., acesulfame-K and sucralose). The sweet taste modulating compound further comprises sweet taste modulators such as enhancers or inhibitors, e.g., enhancers such as Modulasense, EN-336-983-7, SweetGem or SucralGem and any blend or mixture enhancing or inhibiting the sweet taste.

A natively expressed receptor is a receptor which is naturally expressed in a cell and which has not been brought into the cell by stable or transient transfection. An example of such receptor is a G-protein coupled sweet taste receptor comprising or consisting of the subunits T1R2 and T1R3 (T1R2/ T1R3), an umami taste receptor comprising or consisting of the subunits T1R1/T1R3 and a bitter taste receptor comprising or consisting of the subunits of the T2R class of G-protein receptors. In general, taste receptors comprise or consist of T1R1, T1R2 T1R3 and/or T2Rs in different combinations.

A cell according to the present invention is any single cell or group of cells, a cell line, an immortalized cell line or a primary cell or primary cell line directly isolated from a tissue or an organ. An example for such cell is a HEK-293, Caco-2/TC7 or MIN6 cell. Primary cells allow for example testing of natural mutations and variations of the taste receptor to adapt a sweet product for example even to a specific person and patient groups, respectively.

An enzyme complementation system comprises an enzyme which is separated into two or more parts, wherein each enzyme part can be fused to a different compound of a cell for example to different factors of the G-protein coupled receptor pathway. If the enzyme is separated in parts the parts are not active, i.e., not functional, or have only a very low activity; once the parts get together a functional enzyme is formed which is active and the enzyme activity can be measured by any method known to a person skilled in the art. The enzyme complementation system is either stably or transiently expressed in a cell. The principle of such enzyme complementation system is depicted for example in Fig. 2. In embodiments of the present invention one part of the enzyme of the enzyme complementation system is for example fused to beta-arrestin of the taste receptor and another part is for example fused to an endosome, the G-protein or one or more of its subunits. The active enzyme reacts with its substrate and metabolizes the substrate, i.e., one or more different products of the substrate result from the interaction of the enzyme with the substrate which is/are measurable and quantifiable, respectively, and represent a detectable signal. Enzymes of the enzyme complementation system are for example selected from the group consisting of beta-galactosidase (beta-Gal), luciferase (Luc), green fluorescent protein (GFP), yellow fluorescent protein (YFP), ubiquitin, dihydrofolate reductase or beta-lactamase. The level of functional enzyme formed is measured for receptor activation, e.g., corresponding to receptor internalization and can be measured.

When the present invention uses the singular form "a", the plural form is always included.

The present invention refers to a method, e.g., an *in vitro* method, for testing the activation of a G-protein coupled taste receptor. The method comprises the steps of a) providing a cell natively expressing the taste receptor and comprising an enzyme complementation system and b) adding a sweet, umami and/or bitter taste modulating compound to the cell; the cell culture medium is optionally removed before adding the sweet, umami and/or bitter taste compound and the cells are optionally rinsed between these steps. In step c) a substrate for the enzyme, e.g., beta-galactosidase, of the enzyme complementation system is added to the cell and the substrate is reacting with the enzyme when the taste receptor is activated and the substrate provides a signal which is detected and optionally quantified in step d). In some embodiments before adding the substrate in step c), the cells together with the sweet, umami and/or bitter taste modulating compound of step b) are incubated at 14 °C to 37 °C, e.g., at 14 °C, 20 °C, 25 °C, 30 °C, 35 °C or 37 °C for e.g., 1 to 24 h, e.g., for 1 h, 3 h, 5 h, 8 h, 10 h, 12, h, 15 h, 20 h or 24 h. Further, in some embodiments before adding the substrate, the sweet, umami and/or bitter taste modulating compound is optionally removed and the cells are optionally rinsed. In general cells are for example rinsed with any type of buffer or NaCl, e.g., 0.7 % NaCl. The signal of step d) is for example a fluorescence or chemiluminescence signal. Buffers used in the method of the present invention are for example HHBSS and SMUF. An important advantage of the method of the present invention is the high sensitivity regarding the sweet, umami or bitter taste modulating compound as shown for example for sucrose and the sweet taste receptor in Fig. 3, wherein the assay has a sensitivity of 0.5 % for sucrose.

In some embodiments the sweet, umami or bitter taste modulating compound tested in a method or kit of the present invention is dissolved in a Simulated Milk Ultra Filtrate (SMUF) buffer, wherein for example the sweet taste modulating compound is present in a concentration of e.g., about 1, 2, 3, 4, 5, 6, 7, 8, 9, 10, 12, or 15 %. SMUF is regarded as a simplified systems of milk constituents which is useful in research on problems of dairy technology and is for example described by Jenness and Koops, 1962. A stock solution of this buffer consists for example of

| Solution I | $KH_2PO_4$ | 39.5 g |
|---|---|---|
| | $K_3$ citrate $\cdot$ $H_2O$ | 30.0 g |
| | $Na_3$ citrate $\cdot$ $2H_2O$ | 44.8 g |
| | $K_2SO_4$ | 4.5 g |

The compounds are filled up to 500 ml with distilled water and are dissolved in the distilled water.

| Solution II | $K_2CO_3$ | 7.5 g |
|---|---|---|
| | KCl | 15.0 g |

The compounds are filled up to 500 ml with distilled water and are dissolved in the distilled water.

| Solution III | $CaCO_3 \cdot 2H_2O$ | 33.0 g |
| | $MgCO_3 \cdot 6H_2O$ | 16.3 g |

The compounds are filled up to 500 ml with distilled water and are dissolved in the distilled water.

Each of solutions I, II and III is stable for 3 to 6 months. The salt comprising milk = 0.08 M.

For the preparation of 11 SMUF about 0.9 l distilled water is taken and

- 20 ml of solution I,
- 20 ml of solution II, and
- 20 ml of solution III

are added to the distilled water; the volume of the distilled water comprising solutions I to III is filled up to 1 l. The SMUF is stored in the fridge at for example 3, 4 or 5 °C. Before use of the SMUF it has to be warmed up to room temperature and the pH has to be adjusted to 6.8.

An example of the composition of such milk salt solution is shown in table 1:

| Type of ion | Concentration (g/251) | Concentration (mg/100g) |
|---|---|---|
| Kalium | 36.33 | 145 |
| Sodium | 10.51 | 42 |
| Calcium | 9.00 | 36 |
| Magnesium | 1.95 | 7.8 |
| Chloride | 28.73 | 115 |

In some embodiments the sweet, the umami and/or the bitter taste modulating compound is added to the cell(s) in different concentrations and/or different sweet, umami and/or bitter taste modulating compounds are added to the cell(s) to compare their effects.

In some embodiments of the present invention the enzyme of the enzyme complementation system comprises or consists of at least two parts, one part is fused to beta-arrestin of the taste receptor and the other part is fused to another factor of the G-protein coupled receptor pathway of the taste receptor such as an endosome, the G-protein or any of its subunits forming a functional enzyme when the taste receptor is activated. The enzyme complementation system comprises or consists of for example two enzyme parts, wherein one part is fused to beta-arrestin and the other part is fused to an endosome, or wherein one part is fused to beta-arrestin and the other part is fused to the alpha, beta or gamma subunit of the G-protein, or the system comprises or consists of for example three parts if one part is fused to beta-arrestin, a second part is fused to one of the above mentioned subunits of the G-protein and a third part is fused to an endosome, or one part is fused to beta-arrestin and a second and third part of the enzyme are fused to different subunits of the G-protein. In addition or alternatively, parts of the enzyme are fused to factors of the recycling pathway of G-protein coupled receptors such as G-protein coupled receptor kinase, SRC, PLC-beta, GRK, GTP, clathrin or clathrin adapter AP2. Once the different parts of the enzyme come together in close proximity, the functional enzyme is formed which is for example when the activated taste receptor is internalized.

In the present invention cells are used which are natively expressing the taste receptor being a sweet taste receptor comprising the subunits T1R2/T1R3, a umami taste receptor comprising the subunits T1R1/T1R3, or a bitter taste receptor comprising or consisting of the subunits of the T2R class of G-protein receptors and which express the enzyme complementation system stably or transiently. The enzyme complementation system, i.e., the parts of the enzyme are encoded for example by a plasmid, a virus and/or bac clones.

In some embodiments the methods of the present invention are used in combination with other commonly used receptor downstream readouts for example based on $Ca^{2+}$, $Ca^{2+}$ sensitive fluorescent dyes or $Ca^{2+}$ sensitive molecule based methods, or cAMP based methods.

In some embodiments of the invention other G-protein coupled receptors are knocked down or are knocked out in the cell used in the methods of the invention to reduce or delete background noise.

The present invention is further directed to a method, e.g., an *in vitro* method, for screening a sweet taste modulating compound which comprises the steps of a) providing a cell natively expressing a taste receptor, wherein the taste receptor is a sweet taste receptor comprising the subunits T1R2 and T1R3 (T1R2/ T1R3), an umami taste receptor comprising the subunits T1R1 and T1R3 (T1R1/T1R3), or any of the bitter taste receptors (T2Rs), and comprising an enzyme complementation system and b) adding a sweet, umami or bitter taste modulating compound to the cell; the cell culture medium is optionally removed before adding the sweet, umami or bitter taste modulating compound and the cells are optionally rinsed between these steps. In step c) a substrate for the enzyme, e.g., beta-galactosidase, of the enzyme complementation system is added to the cell and the substrate is reacting with the enzyme when the taste receptor is activated and the substrate provides a signal which is detected and optionally quantified in step d). In step c) the sweet, umami or bitter taste modulating compound is optionally removed before the substrate is added and in any step the cells are optionally rinsed with any buffer or NaCl, e.g. 0.7 % NaCl before the sweet, an umami or a bitter taste modulating compound is added, before the substrate is added and/or before the substrate signal is detected, measured and quantified, respectively. In a further step e) the signal(s) detected in step d) is/are referenced to a standard or are compared with each other. The standard is for example prepared with a known natural sugar, a known artificial sugar or a known taste modulator, such as an enhancer and/or inhibitor, or combinations thereof in one or more concentrations. Hence, the method for screening a sweet, an umami or a bitter taste modulating compound is based on the method for testing the activation of a G-protein coupled taste receptor further comprising an evaluation step e).

In embodiments of this screening method the effect of different sweet, umami and/or bitter taste modulating compounds can be compared and/or the effect of a sweet, an umami or a bitter taste modulating compound in different concentrations. In the method or kit of the present invention the G-protein coupled taste receptor is a sweet taste receptor comprising or consisting of the subunits T1R2 and T1R3 (T1R2/ T1R3), a umami taste receptor comprising or consisting of the subunits T1R1 and T1R3 (T1R1/T1R3), or is any of the bitter taste receptors (T2Rs). A sweet taste modulating compound is for example selected from the group consisting of a food product, a beverage, a medicament or an extract thereof, a natural sugar such as lactose, sucrose, glucose, maltose or fructose, an artificial, e.g., non-caloric or low caloric sweetener, a sweet taste modulator such as an enhancer and/or an inhibitor (e.g., enhancers such as Modulasense, EN-336-983-7, SweetGem or SucralGe) or a blend enhancing or inhibiting the sweet taste as above mentioned for the taste method of the present invention.

The method for testing the activation of the G-protein coupled taste receptor and the method for screening a sweet, an umami and/or a bitter taste compound, respectively, of the present invention are suitable for high throughput screening. The skilled person will be able to suitably adapt the screening method of the invention for high throughput screening, based on common general knowledge of methods and equipment therefore.

In addition, the present invention relates to a kit for testing the activation of a G-protein coupled taste receptor or for screening a taste modulating compound comprising a cell natively expressing the taste receptor and stably or transiently expressing an enzyme complementation system, and a substrate for the enzyme of the enzyme complementation system, wherein the receptor is a sweet taste receptor comprising the subunits T1R2 and T1R3 (T1R2/ T1R3), wherein the G-protein coupled taste receptor is an umami taste receptor comprising the subunits T1R1 and T1R3 (T1R1/T1R3), or wherein the G-protein coupled taste receptor is any of the bitter taste receptors (T2Rs). The signal received from the substrate which reacts with the active enzyme of the activated receptor is detected by any method known by a person skilled in the art.

In other embodiments the testing and screening methods of the present invention are used for the testing and screening of sweetness inhibitors or inhibitors of umami or bitter taste. Cells are stimulated by adding a sweet, an umami or bitter taste modulating compound in step a), then the cells are tested and screened according to steps b) to d) of the present methods, wherein the sweet, umami or bitter taste modulating compound in step b) is a sweet, an umami and bitter taste inhibitor, respectively.

**Examples**

**Example 1: Sweet receptor assay technology**

Human Embryonic Kidney (HEK-293) cells were chosen as recipient cell line for the PathHunter™ beta-Arrestin Enzyme Fragment Complementation (EFC) technology (enzyme complementation system) to be able to investigate receptor internalisation events based on transient expression levels of the T1R2/T1R3 receptor. Based on described GPCR internalisation mechanisms (Claing et al 2002; Reiter and Lefkowitz 2006), it was hypothesized that upon T1R2/T1R3-receptor activation by a sweet tasting agonist, i.e., a sweet taste modulating compound, the intracellular C-terminal tail of the receptor will be phosphorylated, increasing the recruitment and binding affinity of beta-arrestin to the receptor (Fig. 2). Binding of beta-arrestin will then stimulate receptor internalisation to the endosome, bringing the EA (enzyme part fused to beta-arrestin) and ProLink (enzyme part fused to the endosome) enzyme parts of beta-gal in close proximity to each other, enabling the formation of a functional beta-gal enzyme. The level of functional beta-Gal formed is a measure for ligand-induced receptor internalisation activity and is quantified by lysing cells using the PathHunter™ substrate mix, allowing the conversion of substrate to chemiluminescence.

PathHunter™HEK293 Active Internalisation Parental cells (HEK-293 cells natively expressing the sweet taste receptor and comprising the enzyme complementation system) were maintained in high glucose Dulbecco's modified Eagle's medium (4.5 g/l D-glucose, L-glutamine, HEPES) supplemented with 10% fetal bovine serum (DMEM-HG) at 37°C, 5% $CO_2$ and 90% humidity. Cells were split once a week using 0.05% Trypsin-EDTA. To select stable clones, cells suspended to a concentration of 13 cells/ml (1.3 cells/100 μl) in DMEM-HG were seeded in clear Poly-D-Lysine coated 96-well plates (CellCoat; Greiner Bio-One GmbH, Frickenhausen, Germany) and incubated for 24 h at 37°C, 5% $CO_2$ and 90% humidity. After 24 h, each well was manually checked for cell density. Wells containing no cells or >1 cells/well were discarded. The remaining wells containing 1 cell/well were maintained until cells became >80% confluent. To expand cells, confluent cell clones were transferred to 6-well TC treated plates. Once expanded, each clone was challenged by 300 mM maltose in low glucose GlutaMAX Dulbecco's modified Eagle's medium (1 g/l D-glucose, GlutaMAX, pyruvate) supplemented with 10% fetal bovine serum (DMEM-LG) according to the beta -arrestin mediated internalisation assay protocol described below. Based on assay performance and observed standard cell morphology and growth rate, five clones were subsequently selected to assess stability of cellular responses during cell passages.

**Example 2: PathHunter™HEK293 Active Internalisation Clone #1057 cells show endogenous expression of the T1R2/T1R3 sweet receptor**

Initial results showed that the custom made PathHunter™HEK293 Active Internalisation Parental cell line displayed enzyme complementation activity in response to various natural sugars in the absence of transfection with T1R2/T1R3 receptor subunits. Since the chemiluminescence signal is only generated in case the EA (enzyme part fused to beta-arrestin) and ProLink™ (enzyme part fused to the endosome) parts of beta-Gal are in close proximity to each other, it was assumed that the observed signal is derived of a sugar sensing receptor internalisation event. This result suggested that the sweet receptor T1R2/T1R3 is endogenously expressed within this cell line which was investigated further. Because the signal intensities in response to sugars within the parental PathHunter™HEK293 Active Internalisation cell pool varied, it was decided to select a stable clone from this primary pool of transformants. From a primary screen of 141 clones which were challenged with 300 mM maltose, five clones were selected to determine stability of cellular responses during cell passages (Fig. 4A). Clone #1057 displayed a sensitive and stable receptor internalisation response over eight passages (Fig. 4B) and was therefore used for further characterization.

For the beta-arrestin mediated internalization assay cells were grown and maintained in DMEM-HG. Once 80% confluent, the cells were glucose-depleted by replacing the medium by DMEM-LG according to Ohta *et al* (2011). After 18 h of glucose starvation, cells were seeded in DMEM-LG at a density of $2.5 \times 10^4$ cells per well in a clear Poly-D-Lysine coated 96-well plate (CellCoat; Greiner Bio-One GmbH, Frickenhausen, Germany) and incubated for 24 h. Next, the PathHunter™ Detection assay kit was used for receptor stimulation according to the manufacturer's instructions. The culture medium was replaced with varying concentrations of the sweet taste modulating compounds, antagonist, modulators or mixtures thereof in either LG-DMEM, HBSS containing 20 mM HEPES (HHBSS) or Simulated Milk Ultra Filtrate (SMUF) buffer (Jenness *et al* 1962) containing GlutaMax and sodium pyruvate. After 3 h of incubation at 20°C, the stimulant was removed and PathHunter™ substrate was added in 100 μl CP10 Cell Plating Reagents. Luminescence was measured using an Infinite F200 (Tecan Group Ltd., Männedorf, Switzerland). Analysis was performed in triplicates (n=3). Data are presented as the mean luminescence change (ΔF) over the base-line level (F):

$$\Delta F/F = (F(\text{stimulant}) \cdot F(\text{negative control}))/F(\text{negative control}).$$

**Example 3: Assay optimisation and validation**

Clone #1057 was used to optimise the PathHunter™ assay performance. First, the optimal duration and incubation temperature of sweet taste modulating compound stimulation was determined. T1R2/T1R3 receptor internalisation activity was highest when sucrose stimulation took place for 3 hours at 20°C (Fig. 5A); alternatively the stimulation took place for 2 or 3 hours at 14 °C, 25 °C, 30 °C and 37 °C, respectively. Next, it was determined whether assay performance is affected by the use of different buffers. Therefore, #1057 cells were stimulated with a sucrose concentration series either dissolved in LG-DMEM (without FBS), HHBSS or Simulated Milk Ultra Filtrate (SMUF) buffer (preparation is described previously). Receptor internalisation responses showed a similar dose-response curve in HHBSS and in LG-DMEM, although maximal responses were slightly higher in HHBSS buffer (Fig. 5B). The dose-response curve indicated a reduced sensitivity using the SMUF buffer although the maximum response observed was in the same range as compared to the other buffers tested. Sucrose-induced receptor internalisation responses in HHBSS and SMUF were significantly reduced by lactisole, a specific inhibitor of the T1R3 receptor subunit (Matholouthi et al 1994; Schiffman et al 1999), indicating that generated chemiluminescence is genuinely caused by internalisation of the sweet taste receptor T1R2/T1R3 (Fig. 5C).

**Example 4: Sweet receptor responses of Clone #1057 to sweet taste modulating compounds**

It was investigated whether different types of sweet taste modulating compounds, could trigger the receptor internalisation pathway as seen for maltose and sucrose. It was found that all sweet taste modulating compounds, i.e., the mono- and disaccharide sugars glucose, fructose, sucrose, lactose and maltose, the sugar alcohols mannitol and maltitol and the artificial sweeteners acesulfame-K and sucralose were able to induce beta-arrestin-mediated receptor internalisation in a concentration-dependent manner (Fig. 6).

Sucrose and fructose displayed a rather slow increase of chemiluminescence signal while for glucose and maltose a steep increase of the internalisation response was observed (Fig. 6A and 6B). T1R2/T1R3-receptor internalisation was initiated at a lower concentration of fructose compared to glucose, although the maximum level of chemiluminescence ($\Delta F/F_{max}$) for both monosaccharides was reached at a concentration of 9%. The $\Delta F/F_{max}$ observed for glucose was 1.2-fold higher than for fructose ($\Delta F/F_{max}$ of 6.2 versus 5.2). While the assay displayed the highest sensitivity to fructose to initiate receptor internalisation, the largest response was observed for maltose (at a concentration of 16.5%) with a $\Delta F/F_{max}$ of 7.2, although it displayed the lowest sensitivity of the natural sugars tested (Fig. 6B). The sugar alcohols mannitol and maltitol showed receptor internalisation responses which were initiated between 2.7 and 3.9% (Fig. 6C). The $\Delta F/F_{max}$-levels reached were nearly equal for both sugar alcohols tested. However, for mannitol this maximal response was reached at a concentration of 7.7% while for maltitol at a concentration of 17.0%. In addition to natural sweet taste modulating compounds, also two artificial sweeteners were tested, i.e., acesulfame-K and sucralose (Fig. 6D). Acesulfame-K displayed a slight reduced T1R2/T1R3 receptor internalisation at concentrations between 0.1 and 2%, while at higher concentrations, acesulfame-K acted as a receptor agonist with maximum responses at 5.5%. However, the $\Delta F/F_{max}$ for acesulfame-K of 1.1 is lower compared to other sweet taste modulating compound. For sucralose, the receptor internalisation response was already observed at the lowest concentration tested, indicating a high sensitivity of the assay for this compound. The largest response was observed at a concentration of 15% with $\Delta F/F_{max}$ levels of 2.9.

**Example 5: Beta-arrestin mediated T1R2/T1R3 receptor internalisation is modulated by sweet enhancers**

To investigate whether boosting of sweet taste by sweetness enhancers is linked to increased levels of beta-arrestin-mediated receptor internalisation, the activity of commercial sweet enhancing modulators (SM) Modulasense (SM1), EN-336-983-7 (SM2), SweetGem (SM3) and SucralGem (SM4) was determined. These modulators were tested for beta-arrestin-mediated receptor internalisation activity on their own as well as in combination with 6, 7, 8 or 9% sucrose (SM1-3) or sucralose (SM4) (Fig. 7).

On their own, the modulators SM1 and SM2 were able to stimulate beta-arrestin-mediated receptor internalisation which was found to be equivalent to 6% sucrose (Fig. 7A). At 0.125%, SM1 enhanced the internalisation response of 6% sucrose to that of an equivalent level of 9.3% sucrose (i.e. an 1.6-fold increase based on sucrose sweetness scale). The modulator SM2 displayed the highest enhancing effect in combination with a 6% sucrose solution which resulted in a maximal 2 fold increase in sweetness at a concentration of 0.25%. The enhancement-effects observed for both SM1 and SM2 were found to be concentration-dependent in the ranges tested. As single stimulants, SM3 and SM4 displayed reduced internalisation responses in the lowest range of concentrations tested. The combinations SM3 with 6% sucrose and SM4 with 6% sucralose both increased receptor internalisation by a factor of 1.2 on a sucrose sweetness scale but interestingly, in both cases these enhancing effects were found not to differ within the concentration range tested (Fig. 7A). Next to the observed enhancement effects when combined with 6% sucrose, SM3 showed enhancer-effects when combined with 9% sucrose but not when combined with 7 or 8% sucrose (Fig. 7C). In contrast, SM2 displayed enhancer effects on all sucrose concentrations tested (Fig. 7B).

The testing matrix of these experiments shows that the optimal enhancing effect is not obtained by combinations of the highest concentrations but the assay allows to determine the optimal blending ratios. The synergistic effects are summarized in the following table 2:

**Table 2:** Synergistic effects of sugar enhancers

| Enhancer | Specific | Single | Combi | Conc. effect |
|---|---|---|---|---|
| EN-336-983-7 | Sucrose | Mild agonist | Effect on 6, 7, 8 and 9% (1.5-1.8x increase); | Yes |
| Sweet Gem | Sucrose | Mild antagonis t | effect on 6% (1.2x) and 9% (1.1x) | No |
| Modulasense | Sucrose | Mild agonist | effect on 6, 7, 8 and 9% (1.2-1.5x increase) | Yes |
| SucralGem | Sucralose | Mild antagonis t | effect on 6% and 9% (1.2x) | Barely |

**Claims**

1. Method for testing the activation of a G-protein coupled taste receptor comprising the steps of:

    a) providing a cell natively expressing the taste receptor and comprising an enzyme complementation system,
    b) adding a taste modulating compound to the cell,
    c) adding a substrate for an enzyme of the enzyme complementation system to the cell, wherein the substrate is reacting with the enzyme, when the taste receptor is activated, providing a signal, and
    d) detecting the signal;

    wherein the G-protein coupled taste receptor is a sweet taste receptor comprising the subunits T1R2 and T1R3 (T1R2/ T1R3), an umami taste receptor comprising the subunits T1R1 and T1R3 (T1R1/T1R3), or any of the bitter taste receptors (T2Rs).

2. Method according to claim 1, wherein the enzyme of the enzyme complementation system comprises at least two parts, one part is fused to beta-arrestin of the taste receptor and the other part is fused to another factor of the G-protein coupled receptor pathway of the taste receptor, the G-protein or any of its subunits forming a functional enzyme when the taste receptor is activated.

3. Method according to claim 2, wherein the functional enzyme is formed, when the activated taste receptor is internalized.

4. Method according to any one of claims 1 to 3, wherein the enzyme of the enzyme complementation system is beta-galactosidase (beta-Gal), luciferase (Luc), green fluorescent protein (GFP), yellow fluorescent protein (YFP), ubiquitin, dihydrofolate reductase or beta-lactamase.

5. Method according to any one of claims 1 to 4, wherein the cell natively expressing the taste receptor is selected from HEK-293, Caco-2/TC7, and MIN6.

6. Method according to any one of claims 1 to 5, wherein the enzyme complementation system is stably or transiently expressed in the cell natively expressing the taste receptor.

7. Method according to any one of claims 1 to 6, wherein the taste modulating compound is a sweet tastant, an umami tastant, a bitter tasting compound, a sweet, umami and/or bitter taste modulator, or a blend modulating the sweet, umami and/or bitter taste

8. Method according to any one of claims 1 to 7, wherein the detectable signal of step d) is a fluorescence or chemiluminescence signal.

9. Method according to any one of claims 1 to 8, wherein in step b) the cell after adding the taste compound is incubated at 14 °C, 20 °C, 25 °C, 30 °C or 37 °C for 2 or 3 h before the substrate in step c) is added.

10. Method for screening a sweet taste modulating compound comprising subjecting this sweet taste modulating compound to a method according to any one of claims 1 to 9, and subjecting the signal or signals detected in step d) to comparison with one or more reference compounds selected from the group consisting of standard compounds, other sweet taste modulating compounds, and/or combinations thereof.

11. Method according to claim 10, wherein the method is suitable for high throughput screening.

12. Method according to claim 10 or 11, wherein the taste modulating compound is a sweet tastant, an umami tastant, a bitter tasting compound, a sweet, umami and/or bitter taste modulator, or a blend modulating the sweet, umami and/or bitter taste.

13. A kit for testing the activation of a G-protein coupled taste receptor or for screening a taste modulating compound comprising a cell natively expressing the taste receptor and stably or transiently expressing an enzyme complementation system, and a substrate for the enzyme of the enzyme complementation system, wherein the receptor is a sweet taste receptor comprising the subunits T1R2 and T1R3 (T1R2/ T1R3), wherein the G-protein coupled taste receptor is an umami taste receptor comprising the subunits T1R1 and T1R3 (T1R1/T1R3), or wherein the G-protein coupled taste receptor is any of the bitter taste receptors (T2Rs).

**Patentansprüche**

1. Verfahren zum Testen der Aktivierung eines G-Protein-gekoppelten Geschmacksrezeptors, umfassend die Schritte:

    a) Bereitstellen einer Zelle, die den Geschmacksrezeptor nativ exprimiert und ein Enzymkomplementationssystem umfasst,
    b) Zugeben einer geschmacksmodulierenden Verbindung zu der Zelle,
    c) Zugeben eines Substrats für ein Enzym des Enzymkomplementationssystems zu der Zelle, wobei das Substrat mit dem Enzym reagiert, wenn der Geschmacksrezeptor aktiviert wird, und ein Signal liefert, und
    d) Erfassen des Signals;

    wobei der G-Protein-gekoppelte Geschmacksrezeptor ein süßer Geschmacksrezeptor mit den Untereinheiten T1R2 und T1R3 (T1R2/T1R3), ein Umami-Geschmackrezeptor mit den Untereinheiten T1R1 und T1R3 (T1R1/T1R3) oder einer der Bittergeschmacksrezeptoren (T2Rs) ist.

2. Verfahren nach Anspruch 1, wobei das Enzym des Enzymkomplementationssystems mindestens zwei Teile umfasst, wobei ein Teil mit Beta-Arrestin des Geschmacksrezeptors fusioniert ist und der andere Teil mit einem anderen Faktor des G-Protein-gekoppelten Rezeptorweges des Geschmacksrezeptors, des G-Proteins oder einer seiner Untereinheiten fusioniert ist, wodurch ein funktionelles Enzym gebildet wird, wenn der Geschmacksrezeptor aktiviert wird.

3. Verfahren nach Anspruch 2, wobei das funktionelle Enzym gebildet wird, wenn der aktivierte Geschmacksrezeptor internalisiert wird.

4. Verfahren nach einem der Ansprüche 1 bis 3, wobei das Enzym des Enzymkomplementationssystems Beta-Galactosidase (beta-Gal), Luciferase (Luc), grün fluoreszierendes Protein (GFP), gelb fluoreszierendes Protein (YFP), Ubiquitin, Dihydrofolatreduktase oder Beta-Lactamase ist.

5. Verfahren nach einem der Ansprüche 1 bis 4, wobei die Zelle, die den Geschmacksrezeptor nativ exprimiert, ausgewählt ist aus HEK-293, Caco-2/TC7 und MIN6.

6. Verfahren nach einem der Ansprüche 1 bis 5, wobei das Enzymkomplementationssystem in der Zelle, die den Geschmacksrezeptor nativ exprimiert, stabil oder vorübergehend exprimiert wird.

7. Verfahren nach einem der Ansprüche 1 bis 6, wobei die geschmacksmodulierende Verbindung ein süßer Geschmacksstoff, ein Umami-Geschmacksstoff, eine bitter schmeckende Verbindung, ein süßer, umami und/oder bitterer Geschmacksmodulator oder eine Mischung ist, die den süßen, den umami und/oder den bitteren Geschmack moduliert.

8. Verfahren nach einem der Ansprüche 1 bis 7, wobei das nachweisbare Signal von Schritt d) ein Fluoreszenz- oder Chemilumineszenzsignal ist.

9. Verfahren nach einem der Ansprüche 1 bis 8, wobei in Schritt b) die Zelle nach Zugabe der Geschmacksverbindung bei 14 °C, 20 °C, 25 °C, 30 °C oder 37 °C 2 oder 3 h lang inkubiert wird, bevor das Substrat in Schritt c) zugegeben wird.

10. Verfahren zum Screenen einer den süßen Geschmack modulierenden Verbindung, umfassend das Unterziehen dieser den süßen Geschmack modulierenden Verbindung einem Verfahren nach einem der Ansprüche 1 bis 9 und das Unterziehen des Signals oder der Signale, die in Schritt d) nachgewiesen worden sind, einem Vergleich mit einer oder mehreren Referenzverbindungen, ausgewählt aus den Gruppe bestehend aus Standardverbindungen, anderen den süßen Geschmack modulierenden Verbindungen und/oder Kombinationen daraus.

11. Verfahren nach Anspruch 10, wobei das Verfahren für das Hochdurchsatz-Screening geeignet ist.

12. Verfahren nach Anspruch 10 oder 11, wobei die geschmacksmodulierende Verbindung ein süßer Geschmacksstoff, ein Umami-Geschmacksstoff, eine bitter schmeckende Verbindung, ein süßer, umami und/oder bitterer Geschmacksmodulator oder eine Mischung ist, die den süßen, den umami und/oder den bitteren Geschmack moduliert.

13. Kit zum Testen der Aktivierung eines G-Protein-gekoppelten Geschmacksrezeptors oder zum Screenen einer geschmacksmodulierenden Verbindung, umfassend eine Zelle, die den Geschmacksrezeptor nativ exprimiert und ein Enzymkomplementationssystem stabil oder vorübergehend exprimiert, und ein Substrat für das Enzym des Enzymkomplementationssystems, wobei der Rezeptor ein süßer Geschmacksrezeptor ist, der die Untereinheiten T1R2 und T1R3 (T1R2/T1R3) umfasst, wobei der G-Protein-gekoppelte Geschmacksrezeptor ein Umami-Geschmacksrezeptor ist, der die Untereinheiten T1R1 und T1R3 (T1R1/T1R3) umfasst, oder wobei der G-Protein-gekoppelte Geschmacksrezeptor einer der Bittergeschmacksrezeptoren (T2Rs) ist.

**Revendications**

1. Procédé pour tester l'activation d'un récepteur du goût couplé à la protéine G comprenant les étapes de :

   a) fourniture d'une cellule exprimant nativement le récepteur du goût et comprenant un système de complémentation enzymatique,
   b) ajout d'un composé de modulation du goût à la cellule,
   c) ajout d'un substrat pour une enzyme du système de complémentation enzymatique à la cellule, le substrat étant mis en réaction avec l'enzyme, lorsque le récepteur du goût est activé, fournissant un signal, et
   d) détection du signal ;
   le récepteur du goût couplé à la protéine G étant un récepteur du goût sucré comprenant les sous-unités T1R2 et T1R3 (T1R2/T1R3), un récepteur du goût umami comprenant les sous unités T1R1 et T1R3 (T1R1/T1R3), ou l'un quelconque parmi les récepteurs du goût amer (T2R).

2. Procédé selon la revendication 1, l'enzyme du système de complémentation enzymatique comprenant au moins deux parties, une partie étant fusionnée à la bêta-arrestine du récepteur du goût et l'autre partie étant fusionnée à un autre facteur de la voie du récepteur couplé à la protéine G du récepteur du goût, la protéine G ou l'une quelconque de ses sous-unités formant une enzyme fonctionnelle lorsque le récepteur du goût est activé.

3. Procédé selon la revendication 2, l'enzyme fonctionnelle étant formée, lorsque le récepteur du goût activé est internalisé.

4. Procédé selon l'une quelconque des revendications 1 à 3, l'enzyme du système de complémentation enzymatique étant la bêta-galactosidase (bêta-Gal), la luciférase (Luc), la protéine fluorescente verte (GFP), la protéine fluorescente jaune (YFP), l'ubiquitine, la dihydrofolate réductase ou la bêta-lactamase.

5. Procédé selon l'une quelconque des revendications 1 à 4, la cellule exprimant nativement le récepteur du goût étant choisie parmi HEK-293, Caco-2/TC7, et MIN6.

6. Procédé selon l'une quelconque des revendications 1 à 5, le système de complémentation enzymatique étant exprimé de manière stable ou de manière transitoire dans la cellule exprimant nativement le récepteur du goût.

7. Procédé selon l'une quelconque des revendications 1 à 6, le composé de modulation du goût étant un agent gustatif sucré, un agent gustatif umami, un composé au goût amer, un modulateur du goût sucré, umami et/ou amer, ou un mélange modulant le goût sucré, umami et/ou amer.

8. Procédé selon l'une quelconque des revendications 1 à 7, le signal détectable de l'étape d) étant un signal de fluorescence ou de chimioluminescence.

9. Procédé selon l'une quelconque des revendications 1 à 8, dans lequel dans l'étape b), la cellule, après l'ajout du composé gustatif, est incubée à 14 °C, 20 °C, 25 °C, 30 °C ou 37 °C pendant 2 ou 3 h avant que le substrat dans l'étape c) ne soit ajouté.

10. Procédé pour le criblage d'un composé modulant le goût sucré comprenant la soumission de ce composé modulant le goût sucré à un procédé selon l'une quelconque des revendications 1 à 9, et la soumission du signal ou des signaux détectés dans l'étape d) à une comparaison avec un ou plusieurs composés de référence choisis dans le groupe constitué par des composés de référence, d'autres composés modulant le goût sucré et/ou des combinaisons correspondantes.

11. Procédé selon la revendication 10, le procédé étant approprié pour un criblage à haut débit.

12. Procédé selon la revendication 10 ou 11, le composé modulant le goût étant un agent gustatif sucré, un agent gustatif umami, un composé au goût amer, un modulateur du goût sucré, umami et/ou amer, ou un mélange modulant le goût sucré, umami et/ou amer.

13. Kit pour tester l'activation d'un récepteur du goût couplé à la protéine G ou pour le criblage d'un composé modulant le goût comprenant une cellule exprimant nativement le récepteur du goût et exprimant de manière stable ou de manière transitoire un système de complémentation enzymatique, et un substrat pour l'enzyme du système de complémentation enzymatique, le récepteur étant un récepteur du goût sucré comprenant les sous-unités T1R2 et T1R3 (T1R2/T1R3), le récepteur du goût sucré couplé à la protéine G étant un récepteur du goût umami comprenant les sous unités T1R1 et T1R3 (T1R1/T1R3), ou le récepteur du goût sucré couplé à la protéine G étant l'un quelconque parmi les récepteurs du goût amer (T2R).

## Fig. 1: Native G-protein coupled taste receptor

John D. Fernstrom, Steven D. Munger, Anthony Sclafani, Ivan E. de Araujo, Ashley Roberts, and Samuel Molinary. Mechanisms for Sweetness . Journal of Nutrition. 2012

## Fig. 2: Schematic presentation of the test methods

## Fig. 3: Assay sensitivity to sucrose

## Fig. 4: Stable clone selection

## Fig. 5: Testing assay conditions

## Fig. 6: Screening of natural sweet taste compounds

## Fig. 7: Screening of sweet taste enhancer

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012178079 A
- US 7927825 B
- WO 2011067202 A
- US 20100120063 A
- WO 2010088633 A
- WO 2004065963 A
- WO 0159451 A
- WO 2005031309 A

**Non-patent literature cited in the description**

- **JOHN D. FERNSTROM ; STEVEN D. MUNGER ; ANTHONY SCLAFANI ; IVAN E. DE ARAUJO ; ASHLEY ROBERTS ; SAMUEL MOLINARY.** Mechanisms for Sweetness. *Journal of Nutrition,* 2012
- **JENNESS R. ; KOOPS, J.** *Netherlands Milk Dairy J.,* 1962, 153-164
- **RUNNING et al.** *Chem. Senses,* 2015, vol. 40 (7), 507-516